# EUROPEAN PATENT SPECIFICATION

(11) **EP 4 398 978 B1**
(45) Date of publication and mention of the grant of the patent: **04.11.2026**
(21) Application number: 22768427.1
(22) Date of filing: 05.09.2022
(51) Int. Cl.: A61N 1/362, A61N 1/37

(54) **IMPLANTABLE MEDICAL DEVICE ALLOWING SAFER DELIVERY OF ATRIAL ANTITACHYCARDIC STIMULATION**
IMPLANTIERBARE MEDIZINISCHE VORRICHTUNG ZUR SICHEREN BEREITSTELLUNG VON ANTITACHYKARDER VORHOFSTIMULATION
DISPOSITIF MÉDICAL IMPLANTABLE PERMETTANT D'ADMINISTRER DE MANIÈRE PLUS SÉCURISÉE LA STIMULATION AURICULAIRE ANTITACHYCARDIQUE

(30) Priority: 09.09.2021 EP 21195672
(43) Date of publication of application: 17.07.2024
(73) Proprietor: BIOTRONIK SE & Co. KG, 12359 Berlin (DE)
(72) Inventor: DOERR, Thomas, 12437 Berlin (DE)
(74) Representative: Biotronik Corporate Services SE
(86) International application number: PCT/EP2022/074603
(87) International publication number: WO 2023/036734

(56) References cited:
- US-A1- 2003 050 563
- US-A1- 2004 015 197
- US-A1- 2013 053 714
- US-A1- 2018 161 572
- US-A1- 2021 170 170

## Description

The present invention relates to an implantable medical device for stimulating a human or animal heart according to claim 1, and to a computer program product according to claim 13.

Implantable medical devices for stimulating a human or animal heart, such as pacemakers, have been known for a long time. They can perform different functions. Among other things, it is known to use such pacemakers for the treatment of atrial arrhythmias. Different stimulation programs can be carried out by an appropriate pacemaker to restore the treated heart to a normal state.

In known implementations, so-called aATP (atrial anti-tachycardia pacing) stimulation pulses are delivered within the atrium of the heart to be stimulated for the termination of atrial tachycardia (AT) or atrial fibrillation (AFib). A temporal synchronization of aATP therapy to a ventricular cardiac action is known.

In further realization examples of aATP therapy, such as described in US 6,876,880 B2, aATP stimulation pulses are delivered when
- rhythm changes of an AT or AFib are detected, or
- when changes in the cycle length of the AT/AFib are detected, or
- when a pre-programmed time has elapsed.

A prerequisite of a safe and reliable atrial antitachycardic pacing is a stably located atrial stimulation electrode. Thus, it is necessary to identify any undesired electrode dislocation prior to deliver atrial antitachycardic pacing pulses.

The known methods for evaluating an atrial electrode position cannot be used or can only be used to a limited extent in case of atrial tachycardia. For example, the evaluation of temporal sequences (e.g., atrial pace / ventricular sense) is not possible because here the atrial tachycardia leads to a non-physiological conduction behavior. Similarly, evaluation of electrode impedances alone is insufficient for such a situation, since an electrode dislocated into the ventricle could also have a valid electrode impedance there.

US 2021/170170 A1 describes a medical device is configured to detect an alternating pattern of signal features determined from consecutive segments of a cardiac electrical signal and determine a gross morphology metric from at least one segment of the cardiac electrical signal. The device is configured to detect cardiac event oversensing in response to detecting the alternating pattern and the gross morphology metric not meeting tachyarrhythmia morphology criteria. The medical device may withhold detecting an arrhythmia in response to detecting the cardiac event oversensing.

It is an object of the present invention to provide a cardiac stimulator that makes delivery of an atrial ATP safer than prior art cardiac stimulators and prevents erroneous ATP delivery.

This object is achieved with an implantable medical device having the features explained in the following. Such an implantable medical device serves for stimulating a human or animal heart. It comprises a processor, a memory unit, a first stimulation unit, a second stimulation unit, a first detection unit, and a second detection unit. The first stimulation unit serves for stimulating an atrium, in particular the right atrium, of a human or animal heart. The second stimulation unit serves for stimulating a ventricle, in particular the right ventricle, of the same heart. The first detection unit serves for detecting an electric signal in the atrium. Finally, the second detection unit serves for detecting an electric signal in the ventricle.

According to an aspect of the present invention, the memory unit comprises a computer-readable program that causes the processor to perform the steps explained in the following when executed on the processor.

First, the first detection unit is used for detecting an electric signal of the heart to be stimulated. This electric signal comprises at least two consecutive amplitudes. Each of the amplitudes correlate with the contraction of the heart to be stimulated. If an electrode of the first detection unit is properly placed within the atrium of the heart to be stimulated, the amplitudes correlate with an atrial contraction of the heart. If, however, a dislocation of the electrode of the first detection unit has been taken place, the amplitudes correlate to both the contraction of the atrium and of the ventricle of the heart to be stimulated. The presently claimed implantable medical device is able to distinguish between these two cases.

For this purpose, it is determined whether a first amplitude of the two consecutive amplitudes and a second amplitude of the two consecutive amplitudes each have a maximum lying within a predetermined intensity range. Alternatively or additionally, it is determined whether a maximum of the first amplitude and a maximum of the second amplitude do not deviate significantly from each other with respect to the intensity

Within the frame of the application the term "a maximum of the first amplitude and a maximum of the second amplitude do not deviate significantly from each other with respect to the intensity" is to be understood as maxima which deviate from each other with respect to intensity by at most 50%, in particular by not mor than 40%, in particular by not more than 30%, in particular by not more than 20 %, in particular by not more than 15 %, in particular not more than 12.5 %, in particular by not more than 10 %, in particular by not more than 7.5 %, in particular by not more than 5 %, in particular by not more than 2.5 %, in particular by not more than 1 % Or maxima which deviate from each other with respect to intensity by at most 2 mV, in particular by not more than 1 mV, in particular by not more than 0.5 mV.

If the maxima of the two amplitudes lie within the predetermined range and/or if the maxima of the two consecutive amplitudes do not significantly deviate from each other with respect to intensity, this is taken as indication that the atrial electrode has not been dislocated, i.e., that it is still appropriate to deliver an atrial antitachycardic pacing. It should be noted in this context that the atrial electrode of the implantable medical device forms part both of the first detection unit and of the first stimulation unit.

If the maxima of both amplitudes do not lie within the predetermined intensity range (if such compliance of the maxima with a given intensity range has been tested at all) and/or if the maxima of the two consecutive amplitudes deviate from each other by more than 20 % (if the maxima of the two amplitudes have been compared with each other at all), an atrial stimulation with the first stimulation unit is prevented. Otherwise, an atrial stimulation with the first stimulation unit is allowed. Thus, the implantable medical device tests if the atrial detection and stimulation electrode is properly kept in place. If this is the case, an atrial stimulation can be performed. If, however, a dislocation of the atrial electrode has been determined, such atrial stimulation is prevented in order to avoid severe health risks of the patient carrying the implantable medical device.

The presently claimed implantable medical device thus ensures a safe implementation of an atrial ATP function, in particular if the device does not have a defibrillation backup. It enables a very reliable dislocation detection of the atrial electrode that can, e.g., be performed immediately before ATP therapy delivery. Then, in the case of an atrial lead dislocated in the ventricle and a resulting over-sensing, an erroneous aATP delivery in the ventricle can be prevented. This is of great physiological importance because aATP delivery in the ventricle could induce ventricular tachycardia or ventricular fibrillation.

In an embodiment, the active implantable medical device is an implantable pulse generator (IPG), an implantable cardioverter-defibrillator (ICD), or a device for cardiac resynchronization therapy (CRT).

In an embodiment, the computer-readable program causes the processor to store the maxima of the amplitudes of the detected electric signal in the memory unit. In doing so, it is possible to compare an actual amplitude maximum with one or more precedingly determined amplitude maxima. Then, a dislocating trend of the atrial electrode can be determined. Thus, a movement of the atrial electrode over time can be easily traced by evaluating the determined amplitude maxima.

In an embodiment, the electric signal comprises a plurality of amplitudes, not only two consecutive amplitudes. Also in such a case, it is particularly easy to detect a dislocation of the atrial electrode over time and to compare the maximum of the current amplitude with not only a single other maximum, but with a plurality of maxima of other amplitudes previously detected with the first detection unit.

In an embodiment, the computer-readable program causes the processor to predetermine the intensity range. This is done on the basis of an average value of at least two previously detected amplitudes of the electric signal. In particular, a plurality (more than two) of previously detected amplitudes is used for calculating such an average.

In an embodiment, the calculated average is a floating average. Thus, the average value is a continuously updated average value that is calculated on the basis of a predetermined number of previously detected amplitudes of the electric signal. To give an example, the predetermined number can be any number in the range of 2 to 2000, in particular of 5 to 1500, in particular of 10 to 1000, in particular of 15 to 900, in particular of 20 to 800, in particular of 30 to 700, in particular of 40 to 600, in particular 50 to 500, in particular of 60 to 400, in particular of 70 to 300, in particular of 80 to 200, in particular of 90 to 100 previously detected amplitudes. This enhances the reliability of a dislocation detection of the atrial electrode since this detection is no longer dependent on a single previously detected value that could be an outlier not having a particular physiological relevance.

In an embodiment, the computer-readable program causes the processor to predetermine the intensity range on the basis of a threshold value and a standard deviation. For this purpose, the threshold value is typically calculated from at least two previously determined values of amplitude maxima, wherein the standard deviation between these at least two values is used for spanning up a confidence interval around the threshold value. It is also possible to add a fixed standard deviation (e.g., in form of a fixed relative or absolute value) to the threshold value. In doing so, a tolerance band can be spanned, wherein a location of the detected amplitude maximum within this tolerance band indicates that the atrial electrode is not dislocated but still in its proper place. On the other hand, if the detected amplitudes lie outside the tolerance band, this indicates a dislocation of the atrial electrode. Such lying outside the tolerance band is used as trigger for preventing an intended subsequent delivery of an atrial stimulation.

In an embodiment, the computer-readable program causes the processor to perform the step of determining whether the maxima of the two consecutive amplitudes lie within a predetermined intensity range and/or whether the maxima of the two amplitudes do not differ significantly from each other as well as the step of preventing an atrial stimulation if the preceding criteria have not been fulfilled and otherwise allowing atrial stimulation with the first stimulation unit are performed once a day. In doing so, a movement course of the atrial electrode can be detected early so that a dislocation of the atrial electrode is also detectable at an early stage. The step of detecting an electric signal comprising the at least two consecutive amplitudes can also be done once a day. Typically, this step is regularly done during a day in order to regularly sense the atrial activity of the heart to be stimulated.

In an embodiment, i) the step of detecting an electric signal, ii) the step of determining whether the maxima of the amplitudes lie within a predeterminable range and/or are in terms of intensity comparable to each other and iii) the step of preventing or allowing an atrial stimulation in dependence on the previous determination step are performed directly before an intended atrial stimulation of the heart with the first stimulation unit. In doing so, this step of delivering an atrial stimulation is made particularly safe since the cardiac state directly prior to the intended stimulation is checked. If the atrial electrode is then not properly placed, the atrial stimulation is prevented.

In an embodiment, the atrial stimulation is an atrial antitachycardic stimulation. As explained above, such an atrial antitachycardic stimulation is intended to terminate an atrial tachycardia or an atrial fibrillation.

In an embodiment, at least one of the first amplitude and the second amplitude reflects a P wave of an atrial contraction of the heart to be stimulated. If both the first amplitude and the second amplitude originate from a P wave of an atrial contraction, both amplitudes should have approximately the same height (i.e., maximum). Then, the atrial electrode is properly located. If, however, one of the first and second amplitudes originates from a P wave and the other amplitude originates from a ventricular signal, the amplitudes will differ with respect to their intensity.

In an embodiment, the computer-readable program causes the processor to combine the above-mentioned conditions (i.e., the first condition according to which the first amplitude and the second amplitude have a maximum lying within a predetermined intensity range and the second condition according to which the maxima of both amplitudes do not deviate significantly from each other in terms of intensity) are combined with at least one further criterion for testing an integrity of an electrode of the first stimulation unit. Then, an atrial stimulation with the first stimulation unit is prevented if at least one of the precedingly mentioned conditions, if tested, and if additionally the at least one further criterion are not fulfilled. Otherwise, an atrial stimulation with the first stimulation unit is allowed. Thus, in this embodiment, the at least one further criterion serves as additional safety net for ensuring a proper placement of the atrial electrode within the atrium of the heart to be stimulated.

In an embodiment, the at least one further criterion is an electrode impedance lying within a predetermined range of 100 - 2000 Ohm, in particular in the range of 150 - 1500 Ohm, in particular in the range of 200 - 1000 Ohm, in particular in the range of 250 - 500 Ohm. If the electrode impedance lies within this range, this is taken as an additional indication that the atrial electrode is properly placed. If, however, the electrode impedance lies without in this range, this is taken as an indication that the atrial electrode is dislocated.

In an aspect, however without being claimed as such, the present disclosure relates to a method for controlling an implantable medical device according to the preceding explanations. This method comprises the steps explained in the following.

First, a first detection unit of the implantable medical device is used for detecting an electric signal of the heart to be stimulated. This electric signal comprises at least two consecutive amplitudes.

Then, it is determined whether a first amplitude of the two consecutive amplitudes and a second amplitude of the two consecutive amplitudes each have a maximum lying within a predetermined intensity range. Alternatively or additionally, it is determined whether a maximum of the first amplitude and a maximum of the second amplitude do not deviate significantly from each other with respect to the intensity. If the maxima of the two amplitudes lie within the predetermined range and/or if the maxima of the two consecutive amplitudes do not significantly deviate from each other with respect to intensity, this is taken as indication that the atrial electrode has not been dislocated, i.e., that it is still appropriate to deliver an atrial antitachycardic pacing.

If the maxima of both amplitudes do not lie within the predetermined intensity range (if such compliance of the maxima with a given intensity range has been tested at all) and/or if the maxima of the two consecutive amplitudes do not deviate significantly from each other (if the maxima of the two amplitudes have been compared with each other at all), an atrial stimulation with a first stimulation unit of the implantable medical device is prevented. Otherwise, an atrial stimulation with the first stimulation unit is allowed.

In an aspect, the present invention relates to computer program product comprising computer-readable code that causes the processor to perform the steps explained in the following when executed on the processor.

First, detecting, with a first detection unit of an implantable medical device, an electric signal of the heart to be stimulated. This electric signal comprises at least two consecutive amplitudes.

Then, it is determined whether a first amplitude of the two consecutive amplitudes and a second amplitude of the two consecutive amplitudes each have a maximum lying within a predetermined intensity range. Alternatively or additionally, it is determined whether a maximum of the first amplitude and a maximum of the second amplitude deviate from each other with respect to the intensity by at most 20 %.

If the maxima of both amplitudes do not lie within the predetermined intensity range (if such compliance of the maxima with a given intensity range has been tested at all) and/or if the maxima of the two consecutive amplitudes do not deviate significantly from each other (if the maxima of the two amplitudes have been compared with each other at all), an atrial stimulation with a first stimulation unit of the implantable medical device is prevented. Otherwise, an atrial stimulation with the first stimulation unit is allowed.

In an aspect, however without being claimed as such, the present disclosure relates to medical method for treating a human or animal patient in need of such treatment. This treatment is done with an implantable medical device for stimulating a patient's heart, in particular with an implantable medical device according to the preceding explanations.

Such an implantable medical device serves for stimulating a human or animal heart. It comprises a processor, a memory unit, a first stimulation unit, a second stimulation unit, a first detection unit, and a second detection unit. The first stimulation unit serves for stimulating an atrium, in particular the right atrium, of the patient's heart. The second stimulation unit serves for stimulating a ventricle, in particular the right ventricle, of the same heart. The first detection unit serves for detecting an electric signal in the atrium. Finally, the second detection unit serves for detecting an electric signal in the ventricle.

The method comprises the steps explained in the following.

First, detecting, with the first detection unit, an electric signal of the heart to be stimulated. This electric signal comprises at least two consecutive amplitudes.

Then, it is determined whether a first amplitude of the two consecutive amplitudes and a second amplitude of the two consecutive amplitudes each have a maximum lying within a predetermined intensity range. Alternatively or additionally, it is determined whether a maximum of the first amplitude and a maximum of the second amplitude do not deviate significantly from each other with respect to their intensity

If the maxima of both amplitudes do not lie within the predetermined intensity range (if such compliance of the maxima with a given intensity range has been tested at all) and/or if the maxima of the two consecutive amplitudes do not deviate significantly from each other (if the maxima of the two amplitudes have been compared with each other at all), an atrial stimulation with a first stimulation unit of the implantable medical device is prevented. Otherwise, an atrial stimulation with the first stimulation unit is allowed.

Finally, an atrial stimulation is performed on the patient's heart if such stimulation is allowed in the precedingly explained step.

All embodiments of the implantable medical device can be combined in any desired way and can be transferred either individually or in any arbitrary combination to the described methods and the described computer program product. Likewise, all embodiments of the described methods can be combined in any desired way and can be transferred either individually or in any arbitrary combination to the respective other method, to the implantable medical device and to the computer program product. Finally, all embodiments described with respect to the computer program product can be combined in any desired way and can be transferred either individually or in any arbitrary combination to the described implantable medical device or to the described methods.

Further details of aspects of the present invention will be explained in the following with respect to exemplary embodiments and accompanying Figures. In the Figures:
- Figure 1: shows a schematic image of correctly positioned atrial and ventricular electrodes;
- Figure 2: shows a dual-chamber electrocardiogram exhibiting an atrial tachycardia with stable P waves;
- Figure 3: shows a schematic image of a dislocated atrial electrode and a correctly positioned ventricular electrode; and
- Figure 4: shows a dual-chamber electrocardiogram exhibiting over-sensing with instable P waves.

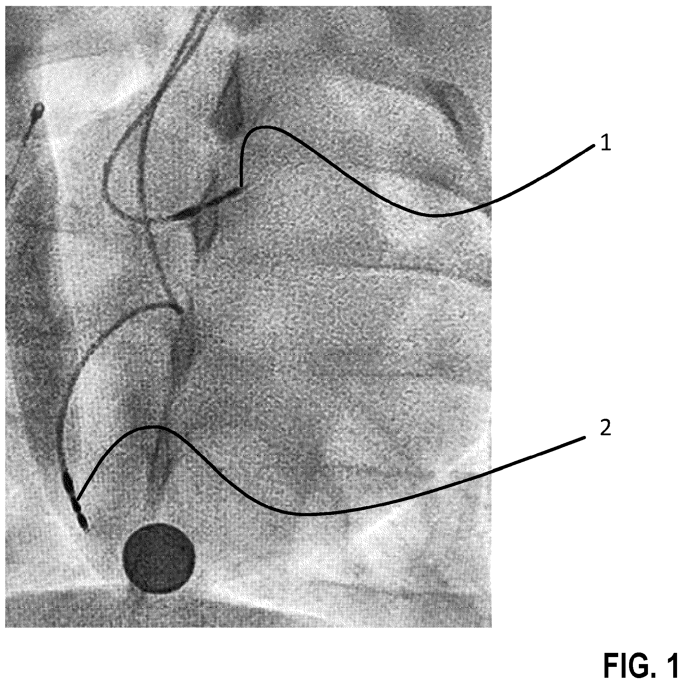

Figure 1 shows a schematic X-ray image of a human heart with a correctly positioned atrial electrode 1 forming part of an atrial detection and stimulation unit of a cardiac pacemaker (i.e., an implantable medical device for stimulating the human or animal heart) as well as with a correctly positioned ventricular electrode 2 forming part of a ventricular detection and stimulation unit of the same pacemaker. The atrial electrode 1 is positioned within the right atrium of the heart. The ventricular electrode 2 is positioned within the apex of the right ventricle of the heart.

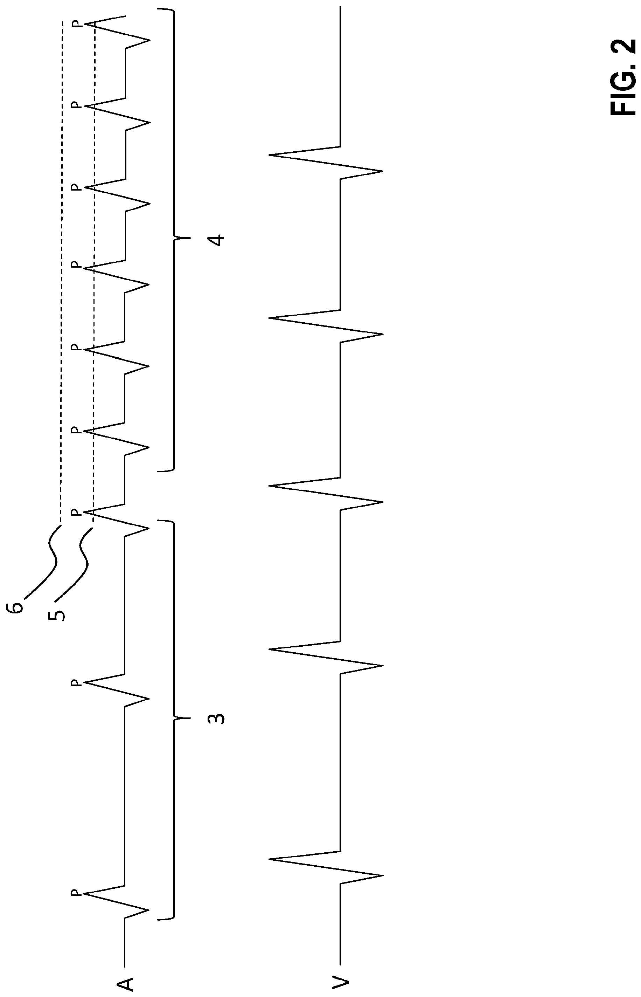

Figure 2 shows a simulated dual chamber electrocardiogram (ECG) with an atrial channel A comprising signals detected with the atrial electrode 1 of Figure 1 and a ventricular channel V comprising signals obtained with the ventricular electrode 2 of Figure 1. In a first portion 3 of the atrial signal A, a sinus rhythm can be observed comprising consecutive P waves P. This sinus rhythm spontaneously converts into an atrial tachycardia in a second portion 4 of the atrial ECG signal A. This atrial tachycardia requires therapy and exhibits constant amplitudes of P waves P.

The maximum of the amplitudes of these P waves P coincide so that a test whether two consecutive maxima deviate from each other by not more than 20 % will be successfully passed. Furthermore, the maxima of the amplitudes of the P waves P lie within a tolerance band spanned between a lower threshold 5 and an upper threshold 6. The tolerance band lies around an average intensity value of the detected P wave amplitudes. It constitutes a predeterminable intensity range.

Thus, also when checking whether the maxima of the amplitudes of the P waves P of the atrial tachycardia observed in the second portion 4 of the atrial ECG signal A lie within the tolerance band, such test would be passed successfully. Consequently, it is apparent from the obtained ECG that the atrial electrode 1 (cf. Figure 1) is correctly positioned within the atrium of the heart to be stimulated.

Since the detected P wave is stable, the criterion for allowing a delivery of an atrial stimulation is fulfilled. Consequently, the implantable medical device is allowed to deliver an atrial antitachycardic pacing pulse in order to terminate the atrial tachycardia.

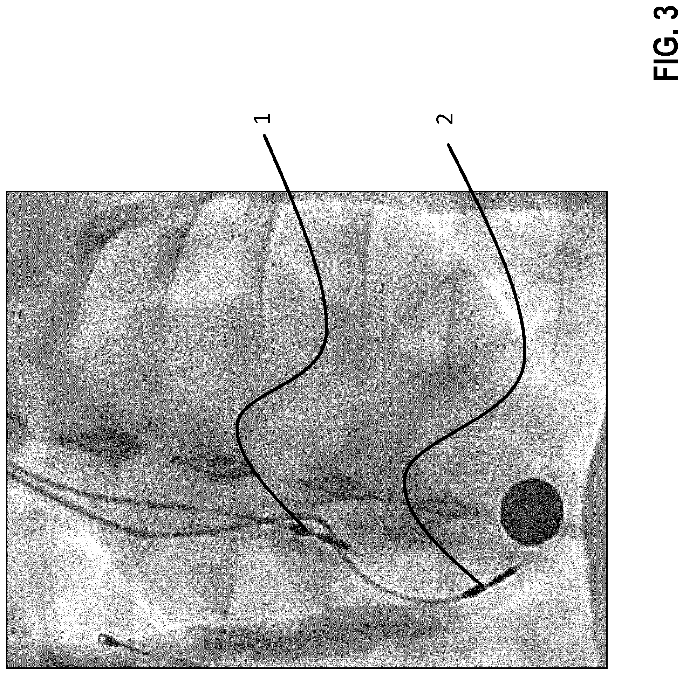

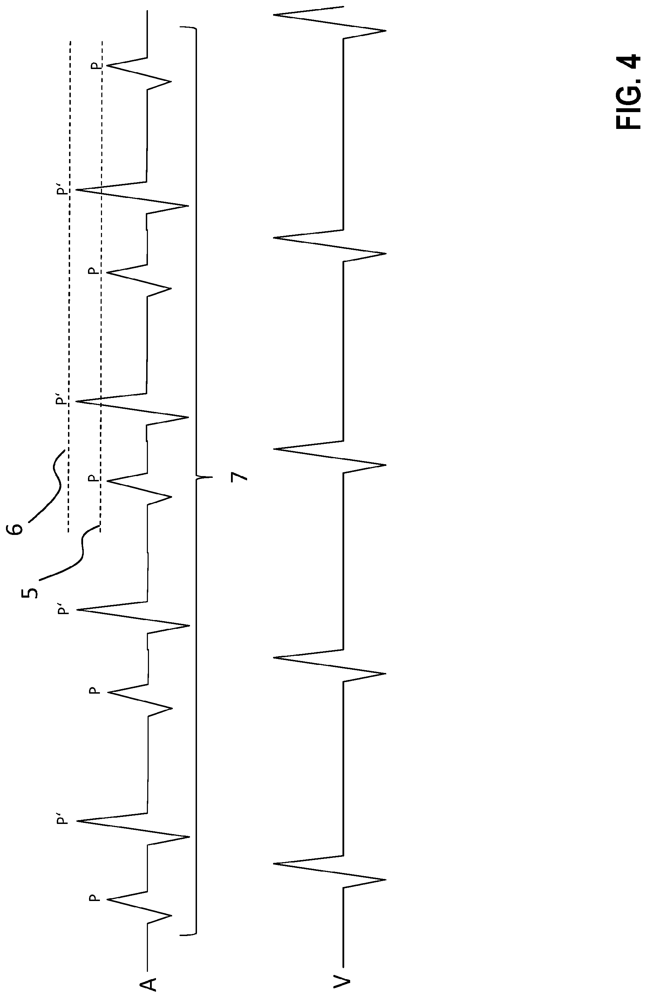

The situation is different in case of a dislocated atrial electrode. This will be explained in more detail referring to Figures 3 and 4.

Figure 3 shows an X-ray image of a human heart with a dislocated atrial electrode 1 and a properly positioned ventricular electrode 2. In this and in all following Figures, the same numeral references will be used for similar elements.

The atrial electrode 1 is now located - due to an undesired dislocation - in the area of the tricuspid valve (i.e., between the right atrium and the right ventricle). The ventricular electrode 2 is correctly positioned within the apex of the right ventricle.

A simulated electrocardiogram (ECG) reflecting identical physiologic conditions as in case of the exemplary embodiment explained with respect Figure 2 is depicted in Figure 4. The signal in the atrial channel A of the ECG only shows a single portion 7 that comprises atrial signals P originating from an atrial P wave as well as ventricular signals P' originating from a ventricular contraction of the heart.

Even though the ventricular signals P' do not originate from P waves, they are considered by the implantable medical device to be the result of an atrial P wave. The cardiac rhythm detection applied by a prior art implantable medical device would recognize in such a case erroneously an atrial tachycardia since the rate of P waves P and apparent P waves P' is too high for a regular sinus rhythm. Consequently, a cardiac pacemaker known from prior art would initiate an atrial antitachycardic pacing therapy. Such a therapy would be connected with the risk to induce a life-threatening ventricular tachyarrhythmia in the ventricle of the heart to be stimulated.

However, when checking the maximum of the amplitudes P and P' of the electric cardiac signal, it is apparent that the P wave signal P does not fall within the tolerance band spanned between the lower threshold 5 and the upper threshold 6. Even though the subsequent ventricular signal P' falls within this tolerance band, the criterion that the maximum of both consecutive amplitudes needs to lie within this range, is not fulfilled. Likewise, by direct comparison of the P wave signal P and the subsequent ventricular signal P', it is apparent that the intensity of the maximum of both amplitudes differs significantly, in particular by more than 20 %. Consequently, also this criterion is not fulfilled.

The implantable medical device according to this embodiment thus prevents the delivery of an atrial stimulation to react on the apparent atrial tachycardia since in fact no atrial tachycardia is present. Rather, the apparent atrial tachycardia is correctly classified as an incorrectly determined atrial tachycardia due to a dislocation of the atrial electrode.

It is apparent from these exemplary embodiments that aspects of the present invention reliably prevent, in case of an undesired dislocation of an atrial electrode of the implantable medical device, a therapy delivery in a cardiac chamber not assigned to this therapy. Aspects of the invention enable the application of an atrial antitachycardic pacing also by cardiac implants without defibrillation function (backup shock function).

## Claims

1. Implantable medical device for stimulating a human or animal heart, comprising a processor, a memory unit, a first stimulation unit configured to stimulate an atrium of a human or animal heart, a second stimulation unit configured to stimulate a ventricle of the same heart, a first detection unit configured to detect an electric signal in the atrium, and a second detection unit configured to detect an electric signal in the ventricle, wherein the memory unit comprises a computer-readable program that causes the processor to perform the following steps when executed on the processor:
a) detecting, with the first detection unit, an electric signal (A) of a heart to be stimulated, the electric signal (A) comprising at least two consecutive amplitudes (P, P') correlating with a contraction of the heart to be stimulated;
b) determining i) whether a first amplitude (P, P') of the two consecutive amplitudes (P, P') and a second amplitude (P, P') of the two consecutive amplitudes (P, P') have a maximum lying within a predeterminable intensity range (5, 6), and/or ii) whether a maximum of the first amplitude (P, P') and a maximum of the second amplitude (P, P') do not deviate significantly from each other in terms of intensity and
c) preventing an atrial stimulation with the first stimulation unit if at least one of conditions i) and ii) of step b), if tested, is not fulfilled, otherwise allowing an atrial stimulation with the first stimulation unit.

2. Implantable medical device according to claim 1, **characterized in that** the computer-readable program causes the processor to store the maxima of the amplitudes (P, P') of the detected electric signal (A) in the memory unit.

3. Implantable medical device according to claim 1 or 2, **characterized in that** the electric signal (A) comprises a plurality of amplitudes (P, P').

4. Implantable medical device according to any of the preceding claims, **characterized in that** the computer-readable program causes the processor to predetermine the intensity range (5, 6) on the basis of an average value of at least two previously detected amplitudes (P, P') of the electric signal.

5. Implantable medical device according to claim 4, **characterized in that** the average value is a continuously updated average value taking into account a predeterminable number of previously detected amplitudes (P, P') of the electric signal (A).

6. Implantable medical device according to any of the preceding claims, **characterized in that** the computer-readable program causes the processor to predetermine the intensity range (5, 6) on the basis of a threshold value and a standard deviation.

7. Implantable medical device according to any of the preceding claims, **characterized in that** the computer-readable program causes the processor to perform steps b) and c) at least once a day.

8. Implantable medical device according to any of the preceding claims, **characterized in that** the computer-readable program causes the processor to perform steps a) to c) directly before an intended atrial stimulation of the heart to be stimulated with the first stimulation unit.

9. Implantable medical device according to any of the preceding claims, **characterized in that** the atrial stimulation is an atrial antitachycardic stimulation.

10. Implantable medical device according to any of the preceding claims, **characterized in that** at least one of the first amplitude (P, P') and the second amplitude (P, P') reflects a P wave (P) of an atrial contraction of the heart to be stimulated.

11. Implantable medical device according to any of the preceding claims, **characterized in that** the computer-readable program causes the processor to combine conditions i) and/or ii) of step b) with at least one further criterion for testing an integrity of an electrode (1) of the first stimulation unit and to prevent an atrial stimulation with the first stimulation unit if at least one of conditions i) and ii) of step b), if tested, and additionally the at least one further criterion is not fulfilled, otherwise allowing an atrial stimulation with the first stimulation unit.

12. Implantable medical device according to any of the preceding claims, **characterized in that** the at least one further criterion is an electrode impedance lying within a predeterminable range.

13. Computer program product comprising computer-readable code that causes a processor to perform the following steps when executed on the processor:
a) detecting, with a first detection unit of an implantable medical device, an electric signal (A) of a heart to be stimulated, the electric signal (A) comprising at least two consecutive amplitudes (P, P') correlating with a contraction of the heart to be stimulated;
b) determining i) whether a first amplitude (P, P') of the two consecutive amplitudes (P, P') and a second amplitude (P, P') of the two consecutive amplitudes (P, P') have a maximum lying within a predeterminable intensity range (5, 6), and/or ii) whether a maximum of the first amplitude (P, P') and a maximum of the second amplitude (P, P') do not deviate significantly from each other in terms of intensity; and
c) preventing an atrial stimulation with a first stimulation unit of the implantable medical device if at least one of conditions i) and ii) of step b), if tested, is not fulfilled, otherwise allowing an atrial stimulation with the first stimulation unit.

## Patentansprüche

1. Implantierbare medizinische Vorrichtung zum Stimulieren eines menschlichen oder tierischen Herzens, umfassend einen Prozessor, eine Speichereinheit, eine erste Stimulationseinheit, die dazu konfiguriert ist, einen Vorhof eines menschlichen oder tierischen Herzens zu stimulieren, eine zweite Stimulationseinheit, die dazu konfiguriert ist, einen Ventrikel desselben Herzens zu stimulieren, eine erste Detektionseinheit, die dazu konfiguriert ist, ein elektrisches Signal in dem Vorhof zu detektieren, und eine zweite Detektionseinheit, die dazu konfiguriert ist, ein elektrisches Signal in dem Ventrikel zu detektieren,
wobei die Speichereinheit ein computerlesbares Programm umfasst, das den Prozessor dazu veranlasst, die folgenden Schritte durchzuführen, wenn es auf dem Prozessor ausgeführt wird:
a) Detektieren, mit der ersten Detektionseinheit, eines elektrischen Signals (A) eines zu stimulierenden Herzens, wobei das elektrische Signal (A) mindestens zwei aufeinanderfolgende Amplituden (P, P') umfasst, die mit einer Kontraktion des zu stimulierenden Herzens korrelieren;
b) Bestimmen, i) ob eine erste Amplitude (P, P') der zwei aufeinanderfolgenden Amplituden (P, P') und eine zweite Amplitude (P, P') der zwei aufeinanderfolgenden (P, P') ein Maximum aufweisen, das sich innerhalb eines vorbestimmbaren Intensitätsbereichs (5, 6) befindet, und/oder ii) ob ein Maximum der ersten Amplitude (P, P') und ein Maximum der zweiten Amplitude (P, P') hinsichtlich der Intensität nicht erheblich voneinander abweichen, und
c) Verhindern einer Vorhofstimulation mit der ersten Stimulationseinheit, wenn mindestens eine der Bedingungen i) und ii) von Schritt b), falls getestet, nicht erfüllt ist, anderenfalls Ermöglichen einer Vorhofstimulation mit der ersten Stimulationseinheit.

2. Implantierbare medizinische Vorrichtung nach Anspruch 1, **dadurch gekennzeichnet, dass** das computerlesbare Programm den Prozessor dazu veranlasst, die Maxima der Amplituden (P, P') des detektierten elektrischen Signals (A) in der Speichereinheit zu speichern.

3. Implantierbare medizinische Vorrichtung nach Anspruch 1 oder 2, **dadurch gekennzeichnet, dass** das elektrische Signal (A) eine Vielzahl von Amplituden (P, P') umfasst.

4. Implantierbare medizinische Vorrichtung nach einem der vorhergehenden Ansprüche, **dadurch gekennzeichnet, dass** das computerlesbare Programm den Prozessor dazu veranlasst, den Intensitätsbereich (5, 6) auf Grundlage eines Durchschnittswerts von mindestens zwei vorher detektierten Amplituden (P, P') des elektrischen Signals vorzubestimmen.

5. Implantierbare medizinische Vorrichtung nach Anspruch 4, **dadurch gekennzeichnet, dass** der Durchschnittswert ein kontinuierlich aktualisierter Durchschnittswert ist, der eine vorbestimmbare Anzahl an vorher detektierten Amplituden (P, P') des elektrischen Signals (A) berücksichtigt.

6. Implantierbare medizinische Vorrichtung nach einem der vorhergehenden Ansprüche, **dadurch gekennzeichnet, dass** das computerlesbare Programm den Prozessor dazu veranlasst, den Intensitätsbereich (5, 6) auf Grundlage eines Schwellenwerts und einer Standardabweichung vorzubestimmen.

7. Implantierbare medizinische Vorrichtung nach einem der vorhergehenden Ansprüche, **dadurch gekennzeichnet, dass** das computerlesbare Programm den Prozessor dazu veranlasst, die Schritte b) und c) mindestens einmal am Tag durchzuführen.

8. Implantierbare medizinische Vorrichtung nach einem der vorhergehenden Ansprüche, **dadurch gekennzeichnet, dass** das computerlesbare Programm den Prozessor dazu veranlasst, die Schritte a) bis c) direkt vor einer beabsichtigten Vorhofstimulation des zu stimulierenden Herzens mit der ersten Stimulationseinheit durchzuführen.

9. Implantierbare medizinische Vorrichtung nach einem der vorhergehenden Ansprüche, **dadurch gekennzeichnet, dass** die Vorhofstimulation eine antitachykarde Vorhofstimulation ist.

10. Implantierbare medizinische Vorrichtung nach einem der vorhergehenden Ansprüche, **dadurch gekennzeichnet, dass** mindestens eine von der ersten Amplitude (P, P') und der zweiten Amplitude (P, P') eine P-Welle (P) einer Vorhofkontraktion des zu stimulierenden Herzens reflektiert.

11. Implantierbare medizinische Vorrichtung nach einem der vorhergehenden Ansprüche, **dadurch gekennzeichnet, dass** das computerlesbare Programm den Prozessor dazu veranlasst, Bedingungen i) und/oder ii) von Schritt b) mit mindestens einem weiteren Kriterium zum Testen einer Integrität einer Elektrode (1) der ersten Stimulationseinheit zu kombinieren und eine Vorhofstimulation mit der ersten Stimulationseinheit zu verhindern, wenn mindestens eine der Bedingungen i) und ii) von Schritt b), falls getestet, und zusätzlich das mindestens eine weitere Kriterium nicht erfüllt sind, anderenfalls Ermöglichen einer Vorhofstimulation mit der ersten Stimulationseinheit.

12. Implantierbare medizinische Vorrichtung nach einem der vorhergehenden Ansprüche, **dadurch gekennzeichnet, dass** das mindestens eine weitere Kriterium darin besteht, dass sich eine Elektrodenimpedanz innerhalb eines vorbestimmbaren Bereichs befindet.

13. Computerprogrammprodukt, umfassend computerlesbaren Code, der einen Prozessor dazu veranlasst, die folgenden Schritte durchzuführen, wenn er auf dem Prozessor ausgeführt wird:
a) Detektieren, mit einer ersten Detektionseinheit einer implantierbaren medizinischen Vorrichtung, eines elektrischen Signals (A) eines zu stimulierenden Herzens, wobei das elektrische Signal (A) mindestens zwei aufeinanderfolgende Amplituden (P, P') umfasst, die mit einer Kontraktion des zu stimulierenden Herzens korrelieren;
b) Bestimmen, i) ob eine erste Amplitude (P, P') der zwei aufeinanderfolgenden Amplituden (P, P') und eine zweite Amplitude (P, P') der zwei aufeinanderfolgenden (P, P') ein Maximum aufweisen, das sich innerhalb eines vorbestimmbaren Intensitätsbereichs (5, 6) befindet, und/oder ii) ob ein Maximum der ersten Amplitude (P, P') und ein Maximum der zweiten Amplitude (P, P') hinsichtlich der Intensität nicht erheblich voneinander abweichen; und
c) Verhindern einer Vorhofstimulation mit einer ersten Stimulationseinheit der implantierbaren medizinischen Vorrichtung, wenn mindestens eine der Bedingungen i) und ii) von Schritt b), falls getestet, nicht erfüllt ist, anderenfalls Ermöglichen einer Vorhofstimulation mit der ersten Stimulationseinheit.

## Revendications

1. Dispositif médical implantable destiné à stimuler un cœur humain ou animal, comprenant un processeur, une unité de mémoire, une première unité de stimulation configurée pour stimuler un atrium d'un cœur humain ou animal, une seconde unité de stimulation configurée pour stimuler un ventricule du même cœur, une première unité de détection configurée pour détecter un signal électrique dans l'atrium, et une seconde unité de détection configurée pour détecter un signal électrique dans le ventricule, dans lequel
l'unité de mémoire comprend un programme lisible par ordinateur qui amène le processeur à réaliser les étapes suivantes lorsqu'elles sont exécutées sur le processeur :
a) détecter, avec la première unité de détection, un signal électrique (A) d'un cœur à stimuler, le signal électrique (A) comprenant au moins deux amplitudes consécutives (P, P') corrélées avec une contraction du cœur à stimuler ;
b) déterminer i) si une première amplitude (P, P') parmi les deux amplitudes consécutives (P, P') et une deuxième amplitude (P, P') parmi les deux amplitudes consécutives (P, P') ont un maximum situé au sein d'une plage d'intensité pouvant être prédéterminée (5, 6), et/ou ii) si un maximum de la première amplitude (P, P') et un maximum de la deuxième amplitude (P, P') ne dévient pas significativement l'un de l'autre en termes d'intensité et
c) empêcher une stimulation atriale avec la première unité de stimulation si au moins l'une des conditions i) et ii) de l'étape b), lorsqu'elle est testée, n'est pas remplie, et autrement autoriser une stimulation atriale avec la première unité de stimulation.

2. Dispositif médical implantable selon la revendication 1, **caractérisé en ce que** le programme lisible par ordinateur amène le processeur à stocker les maximums des amplitudes (P, P') du signal électrique détecté (A) dans l'unité de mémoire.

3. Dispositif médical implantable selon la revendication 1 ou 2, **caractérisé en ce que** le signal électrique (A) comprend une pluralité d'amplitudes (P, P').

4. Dispositif médical implantable selon l'une quelconque des revendications précédentes, **caractérisé en ce que** le programme lisible par ordinateur amène le processeur à prédéterminer la plage d'intensité (5, 6) sur la base d'une valeur moyenne d'au moins deux amplitudes précédemment détectées (P, P') du signal électrique.

5. Dispositif médical implantable selon la revendication 4, **caractérisé en ce que** la valeur moyenne est une valeur moyenne actualisée en continu prenant en compte un nombre pouvant être prédéterminée d'amplitudes (P, P') précédemment détectées du signal électrique(A).

6. Dispositif médical implantable selon l'une quelconque des revendications précédentes, **caractérisé en ce que** le programme lisible par ordinateur amène le processeur à prédéterminer la plage d'intensité (5, 6) sur la base d'une valeur de seuil et d'un écart type.

7. Dispositif médical implantable selon l'une quelconque des revendications précédentes, **caractérisé en ce que** le programme lisible par ordinateur amène le processeur à réaliser les étapes b) et c) au moins une fois par jour.

8. Dispositif médical implantable selon l'une quelconque des revendications précédentes, **caractérisé en ce que** le programme lisible par ordinateur amène le processeur à réaliser les étapes a) à c) directement avant une stimulation atriale souhaitée du cœur à stimuler avec la première unité de stimulation.

9. Dispositif médical implantable selon l'une quelconque des revendications précédentes, **caractérisé en ce que** la stimulation atriale est une stimulation atriale antitachycardique.

10. Dispositif médical implantable selon l'une quelconque des revendications précédentes, **caractérisé en ce qu'**au moins une parmi la première amplitude (P, P') et la deuxième amplitude (P, P') réfléchit une onde P (P) d'une contraction atriale du cœur à stimuler.

11. Dispositif médical implantable selon l'une quelconque des revendications précédentes, **caractérisé en ce que** le programme lisible par ordinateur amène le processeur à combiner les conditions i) et/ou ii) de l'étape b) avec au moins un critère supplémentaire pour tester une intégrité d'une électrode (1) de la première unité de stimulation et à empêcher une stimulation atriale avec la première unité de stimulation si au moins l'une des conditions i) et ii) de l'étape b), lorsqu'elle est testée, et de plus l'au moins un critère supplémentaire n'est pas rempli, et autrement autoriser une stimulation atriale avec la première unité de stimulation.

12. Dispositif médical implantable selon l'une quelconque des revendications précédentes, **caractérisé en ce que** l'au moins un critère supplémentaire est une impédence d'électrode située au sein d'une plage pouvant être prédéterminée.

13. Produit de programme informatique comprenant un code lisible par ordinateur qui amène un processeur à réaliser les étapes suivantes lorsqu'elles sont exécutées sur le processeur :
a) détecter, avec une première unité de détection d'un dispositif médical implantable, un signal électrique (A) d'un cœur à stimuler, le signal électrique (A) comprenant au moins deux amplitudes consécutives (P, P') corrélées avec une contraction du cœur à stimuler ;
b) déterminer i) si une première amplitude (P, P') parmi les deux amplitudes consécutives (P, P') et une deuxième amplitude (P, P') parmi les deux amplitudes consécutives (P, P') ont un maximum se trouvant au sein d'une plage d'intensité pouvant être prédéterminée (5, 6), et/ou ii) si un maximum de la première amplitude (P, P') et un maximum de la deuxième amplitude (P, P') ne dévient pas significativement l'un de l'autre en termes d'intensité ; et
c) empêcher une stimulation atriale avec une première unité de stimulation du dispositif médical implantable si au moins l'une des conditions i) et ii) de l'étape b), lorsqu'elle est testée, n'est pas remplie, et autrement autoriser une stimulation atriale avec la première unité de stimulation.
